# EUROPEAN PATENT APPLICATION

(11) **EP 4 046 567 A1**
(43) Date of publication of application: **24.08.2022**
(21) Application number: 22156557.5
(22) Date of filing: 14.02.2022
(51) Int. Cl.: A61B 3/16

(54) **SYSTEM FOR NON-CONTACT MEASURING INTRAOCULAR PRESSURE OF AN EYE**

(30) Priority: 22.02.2021 PL 43708521
(71) Applicant: Frey Spolka Jawna, 05-502 Piaseczno (PL)
(72) Inventor: Frey, Jacek, 05-502 Piaseczno (PL); Frey, Wojciech, 05-502 Piaseczno (PL)
(74) Representative: Wlasienko, Jozef

(57) **Abstract**

The subject of the invention is a tonometer system for measuring an intraocular pressure using the "air puff' non-contact method, wherein the tonometer system comprises at least an applanation signal detection system consisting of a light source (09) illuminating the cornea of an examined eye (21) and a receiver (10) receiving the light reflected from the cornea that enables the reflected light beam intensity to be measured, an applanation pulse generating system consisting of a piston (05A) arranged in a cylinder (05) and driven by an actuator (06), a compression chamber (03) connected to the applanation pulse generating system through an air channel (07A), an air nozzle (18) by means of which the air compressed in the compression chamber is directed at the cornea of the examined eye, a pressure sensor (08) used for reading an instantaneous pressure value in the compression chamber, an electronic control system (04) that reads from the sensors (08, 10) and analyses the pressure value in the compression chamber (03), the intensity value of the light beam reflected from the cornea of the eye and controls the movement of the piston (05A), wherein the system further comprises an electrically operated valve (07) with at least two positions, arranged between the applanation pulse generating system (05, 05A, 06) and the compression chamber (03) controlled by the electronic control system (04) such that in one of the positions of the valve (07) a free air flow between the applanation pulse generating system (05, 05A, 06) and the compression chamber (03) is possible, and in the other position of the valve (07) the air flow between the applanation pulse generating system (05, 05A, 06) and the compression chamber (03) is not possible.

## Description

The subject of the invention is an "air puff' type non-contact tonometer system for measuring an intraocular pressure of an eye using a non-contact method that is characterized by increased resistance to biological contamination and improves patient comfort.

### Summary of the invention

According to the invention there is provided a system for measuring an intraocular pressure using a non-contact method (air puff tonometer) consisting in the intensity detection of a light beam reflected from the eye's cornea which is deformed by a compressed air pulse (an applanation pulse), wherein the system comprises an applanation pulse generator consisting of a piston arranged in a cylinder and an electric actuator driving the piston, a compression chamber connected to the applanation pulse generator, a valve enabling the air flow between the applanation pulse generator and the compression chamber to be controlled, an air pressure detector in the compression chamber, a nozzle directing the compressed air stream towards the examined eye, a measuring light beam projector together with a detector of the light intensity reflected from the cornea, an electronic driver of the device, which during the measuring cycle constantly reads and analyses the pressure value in the compression chamber and the intensity value of the light reflected from the cornea of the eye, and based on the read information controls the operation of the applanation pulse generator and controls the valve controlling the air flow between the compression chamber and the applanation pulse generator such that upon reaching a predetermined pressure value in the compression chamber it switches the valve from a position enabling the free air flow from the applanation pulse generator to the compression chamber to a position in which such flow is not possible, wherein the intraocular pressure value is calculated by an electronic driver of the device based on the read pressure values in the compression chamber and the intensity of the light beam reflected from the cornea, and the result is displayed on a connected display.

Preferably, the valve controlling the air flow switched to the state of blocking the flow between the compression chamber and the applanation pulse generator enables a controlled air outflow from the applanation pulse generator to the environment.

Preferably, a time point of switching the valve controlling the air flow is determined based on the pressure in the compression chamber.

Preferably, an additional system is provided, which constantly pumps the air to the compression chamber in order to provide the air pressure inside the compression chamber higher than the air pressure of the environment.

Preferably, the control system is provided with a capability of reading the piston position or the position of the electric actuator that drives the piston in order to control the piston movement such that its movement speed can be controlled.

Preferably, an additional optical system is provided, which enables a non-contact measurement of corneal thickness of the examined eye.

Preferably, the electronic control system allows the operation in several measuring ranges so as to adjust the applanation pulse energy to the selected measuring range.

Preferably, additional optical marks are provided, which enable a precise automatic approach of a tonometer measuring head to the examined eye.

### Brief description of the drawings

The invention and solutions known in the prior art are presented in the drawing in which:
Fig. 1 shows a system for non-contact measuring an intraocular pressure according to the present invention.
Fig. 2 shows the relationship between the deformation of the cornea of the examined eye, the pressure value in the compression chamber and the intensity value of the light beam reflected from the cornea in a typical measurement cycle on the non-contact tonometer.

### Method for measuring an intraocular pressure in "air puff" type non-contact tonometers

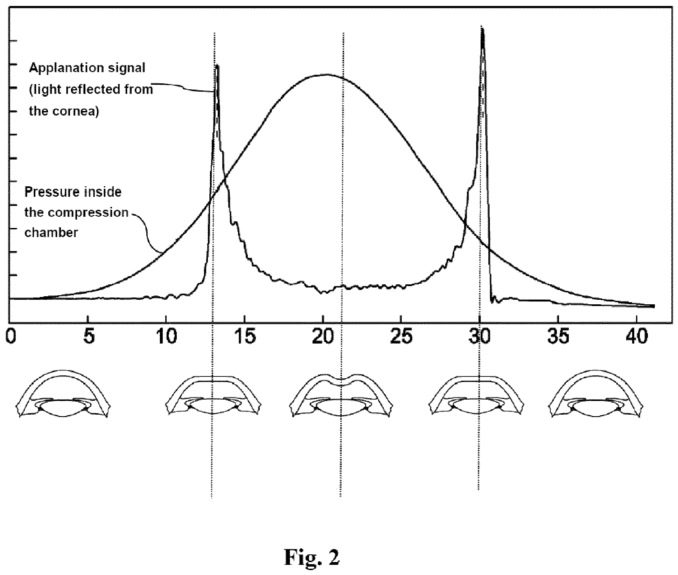

The principle of measuring an intraocular pressure using an "air puff' type non-contact tonometer is presented in Fig. 2. The eye to be measured is positioned in front of an air nozzle of the tonometer and illuminated with a measuring light beam, most often an infrared light beam, whereas a reading system records the intensity of the light reflected from the corneal surface. Once the measurement is triggered, a compressed air pulse (an applanation air pulse) is directed towards the eye by the air nozzle, which causes the corneal surface to flatten and increases its light- reflecting surface, and the detector records an increased energy of the reflected light beam (applanation signal). Various illumination and light beam measurement systems are used, the light beam incidenting perpendicularly to the corneal plane along the air nozzle axis or at an angle to the air nozzle axis.

During the measurement cycle, the cornea of the eye is deformed and goes through successive states, from the rest state a), the applanation state b), the concave state c) and the reapplanation state d) and the rest state e). The duration of the entire cycle is very short and depending on the device design and the pressure inside the eyeball, it may last from several to several dozen milliseconds.

The estimation of the intraocular pressure is performed based on the analysis and the recording of the pressure value inside the compression chamber during the measuring cycle and on the corresponding applanation signal curve value.

While the cornea deformed by the applanation pulse is returning to the rest state, particles of tear fluid, epithelium, and other biological substances on the surface of the cornea are ejected towards the air nozzle. This phenomenon causes contamination of the measuring device. Moreover, in most solutions of "air puff' type tonometers, the piston of the applanation pulse generating system returning to the rest position produces the reverse movement of air in the nozzle, which sucks the particles ejected from the eye surface to the compression chamber. This leads to the contamination of the entire device and to the possible transmission of biological infections to subsequent examined patients.

### Description of the system according to the invention

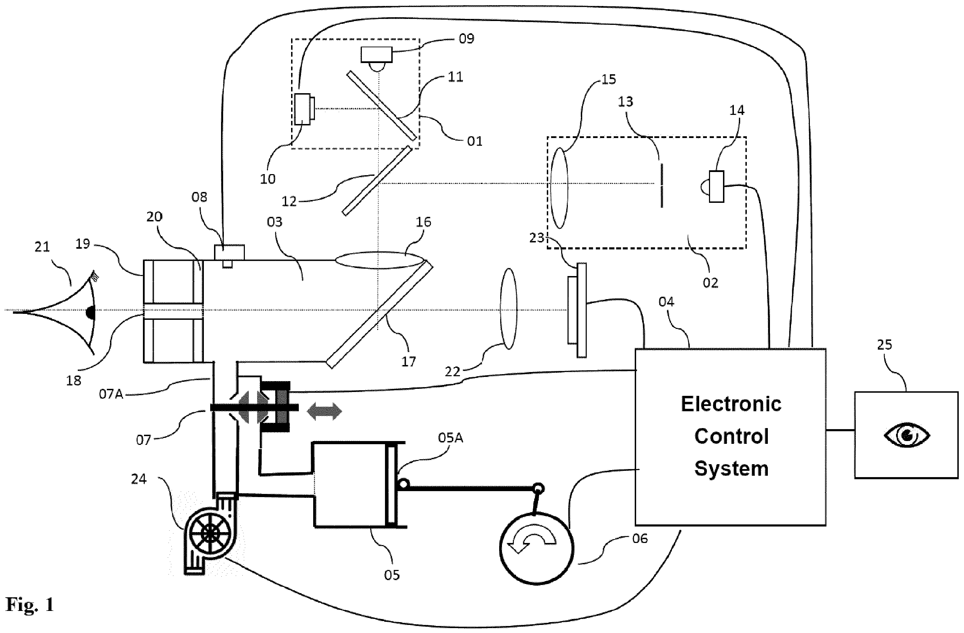

A description of the system according to the present invention is shown in Fig. 1. A light beam illuminating system 01 for illuminating an eye is composed of a light source 09, a detector 10 of the light energy reflected from the cornea, a light beam splitter 11; during the measurement, the examined eye 21 is directed towards an indicator generated in a fixator system 02 composed of a light source 14, a diaphragm 13 and a lens 15; light beams from the light beam illuminating system and the fixator system are directed by a light beam splitter 12, a lens 16 and a further light beam splitter 17 towards the examined eye 21. An applanation pulse generating system is composed of a piston arranged in a cylinder 05 and an actuator 06 that drives the piston 05A. Air compressed in the applanation pulse generating system is directed to a compression chamber 03 through an inlet channel 07A, in which an electronically controlled bistable cut-off valve 07 is arranged. A pressure sensor 08 is used for measuring the pressure value in the compression chamber 03, from which the compressed air is directed towards the examined eye 21 by means of a nozzle 18 mounted in two transparent glass windows 19 and 20. During the measuring cycle, an electronic control system 04 constantly reads and records the pressure in the compression chamber 03 from the sensor 08, the light intensity of the beam reflected from the cornea of the eye from the sensor 10, and additionally the instantaneous speed and the position of the piston 05A, if the piston 05A or the electric actuator 06 are equipped with a position reading system. Based on the read data, the electronic control system 04 controls the movement speed of the piston 05A and the position of the valve 07. An eye preview camera composed of a sensor 23 and a lens 22 records an image of the anterior part of the eye in order to ensure a proper setting of the non-contact tonometer in the measuring position. A display 25 is used for presenting the image from the eye preview camera and for displaying the measurement results. An air pump 24 controlled by the electronic control system 04 has the purpose of producing the pressure in the compression chamber higher than the atmospheric pressure, which ensures the constant air flow from the compression chamber outside through the nozzle 18 and hinders the ingress of impurities to the compression chamber. The air flow forced by the pump 24 has to be very small so that it is not felt by the patient in the eye.

At the beginning of the measuring cycle, the valve 07 is set to the position enabling a free air flow from the applanation pulse generating system to the compression chamber. The movement of the piston 05A causes the pressure in the compression chamber 03 to build up. Upon reaching a predetermined pressure level in the chamber 03 and stored in the electronic control system 04, the electronic control system switches the valve 07 in the position preventing the further air inflow to the compression chamber. The pressure value for which the valve is switched must be chosen such that the energy accumulated in the compression chamber is sufficient for complete flattening the cornea of the examined eye. After switching the valve 07 in the position blocking the air flow from the applanation pulse generating system to the compression chamber, the electronic control system stops the piston movement and allows it to return to its original position (the actuator usually comprises a return spring). While the piston is returning, the valve 07 prevents the air from being sucked from the compression chamber, and therefore there is no return air flow through the nozzle 18 and no suction of impurities to the compression chamber.

The valve 07 can be designed such that in the first position (open) only the free air flow from the cylinder 05 to the compression chamber 03 is possible, and in the second position (closed) the air flow from the cylinder 05 to the compression chamber 03 is impossible, but a small air flow to the environment is possible. This allows the piston 05A movement to slow down slowly, and then allows the air to be sucked into the cylinder 05 while the piston 05A is returning to the rest position.

As a result of such design, the "air puff' non-contact applanation tonometer system is largely protected against the ingress of contaminants into the device, and thus the risk of infections in the tested patients is reduced. Additionally, with a proper selection of the width of air channels connecting the applanation pulse generating system and the compression chamber 03 and the pressure for which the valve 07 is switched, it is possible to significantly reduce the patient's perception of the applanation impulse.

## Claims

1. A tonometer system for measuring an intraocular pressure using the "air puff' non-contact method, wherein the tonometer system comprises at least an applanation signal detection system consisting of a light source (09) illuminating the cornea of an examined eye (21) and a receiver (10) receiving the light reflected from the cornea that enables the reflected light beam intensity to be measured, an applanation pulse generating system consisting of a piston (05A) arranged in a cylinder (05) and driven by an actuator (06), a compression chamber (03) connected to the applanation pulse generating system through an air channel (07A), an air nozzle (18) by means of which the air compressed in the compression chamber is directed at the cornea of the examined eye, a pressure sensor (08) used for reading an instantaneous pressure value in the compression chamber, an electronic control system (04) that reads from the sensors (08, 10) and analyses the pressure value in the compression chamber (03), the intensity value of the light beam reflected from the cornea of the eye and controls the movement of the piston (05A), **characterized in that** the system further comprises:
an electrically operated valve (07) with at least two positions, arranged between the applanation pulse generating system (05, 05A, 06) and the compression chamber (03), controlled by the electronic control system (04) such that in one of the positions of the valve (07) a free air flow between the applanation pulse generating system (05, 05A, 06) and the compression chamber (03) is possible, and in the other position of the valve (07) the air flow between the applanation pulse generating system (05, 05A, 06) and the compression chamber (03) is not possible;

2. The system according to claim 1 **characterized in that** the time point of switching the valve (7) from the position enabling the air flow between the applanation pulse generating system (05, 05A, 06) and the compression chamber (03) to the position preventing such air flow, is determined based on the pressure value in the compression chamber (03).

3. The system according to claim 1 or 2 **characterized in that** the piston (05A) or the actuator (06) comprises a position reading system, by means of which the electronic control system (04) can determine the position and the movement speed of the piston (05A) at any time.

4. The system according to claim 1 - 3 **characterized in that** the system comprises an additional pump (24) delivering the air to the compression chamber (03).
